(19)

**Europäisches Patentamt**
**European Patent Office**
**Office européen des brevets**

(11) **EP 4 221 735 B1**

(12) **FASCICULE DE BREVET EUROPEEN**

(45) Date de publication et mention
de la délivrance du brevet:
**10.09.2025 Bulletin 2025/37**

(21) Numéro de dépôt: **21787315.7**

(22) Date de dépôt: **27.09.2021**

(51) Classification Internationale des Brevets (IPC):
***A61K 36/87*** *(2006.01)*   ***A61K 31/05*** *(2006.01)*
***A61P 7/02*** *(2006.01)*   ***A61P 17/00*** *(2006.01)*
***A61P 25/00*** *(2006.01)*   ***A61K 8/49*** *(2006.01)*

(52) Classification Coopérative des Brevets (CPC):
(C-Sets disponibles)
**A61P 7/02; A61K 8/347; A61K 8/498;**
**A61K 8/9789; A61K 8/9794; A61K 31/05;**
**A61K 31/353; A61K 36/87; A61P 17/00;**
**A61P 25/00; A61Q 19/00;** A61K 2800/92   (Cont.)

(86) Numéro de dépôt international:
**PCT/EP2021/076536**

(87) Numéro de publication internationale:
**WO 2022/069416 (07.04.2022 Gazette 2022/14)**

(54) **COMPOSITION COMPRENANT DES MONOMERES DE FLAVANOL ET DE L'E-VINIFERINE**

ZUSAMMENSETZUNG MIT FLAVANOL UND E-VINIFERIN-MONOMEREN

COMPOSITION COMPRISING FLAVANOL AND E-VINIFERIN MONOMERS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **29.09.2020 FR 2009924**

(43) Date de publication de la demande:
**09.08.2023 Bulletin 2023/32**

(73) Titulaire: **Activ'Inside**
**33750 Beychac-et-Caillau (FR)**

(72) Inventeurs:
• **GAUDOUT, David**
**33360 CARIGNAN DE BORDEAUX (FR)**
• **REY, Stéphane**
**26200 MONTELIMAR (FR)**
• **LEMAIRE, Benoit**
**33500 LIBOURNE (FR)**
• **POURTAU, Line**
**33610 CESTAS (FR)**
• **POUCHIEU, Camille**
**33410 GABARNAC (FR)**
• **DUBREUIL, Séverine**
**44119 TREILLIERES (FR)**

• **MORAS, Benjamin**
**33270 FLOIRAC (FR)**
• **BORNERIE, Mégane**
**33600 PESSAC (FR)**
• **GABASTON, Julien**
**33140 VILLENAVE D'ORNON (FR)**
• **ROLAIT, Manon**
**33500 ARVEYRES (FR)**
• **DE VULPILLIERES, Astrid**
**33000 BORDEAUX (FR)**

(74) Mandataire: **Aquinov**
**12, Cours Xavier Arnozan**
**33000 Bordeaux (FR)**

(56) Documents cités:
**FR-A1- 3 042 712   US-A1- 2018 271 928**

• **GYÖNGYI NÉMETH ET AL: "Stilbenes in the
different organs of Vitis vinifera cv. Merlot
grafted on TK5BB rootstock", vol. 51, no. 3, 26
September 2017 (2017-09-26), pages 323 - 328,
XP055799260, Retrieved from the Internet
<URL:http://oeno-one.eu/article/viewFile/1861/
xml> DOI: 10.20870/oeno-one.2017.51.4.1861**

- **G.K. JAYAPRAKASHA ET AL: "Antioxidant activity of grape seed (Vitis vinifera) extracts on peroxidation models in vitro", FOOD CHEMISTRY, vol. 73, no. 3, 1 May 2001 (2001-05-01), NL, pages 285 - 290, XP055324750, ISSN: 0308-8146, DOI: 10.1016/S0308-8146(00)00298-3**
- **BENSALEM JULIEN ET AL: "Polyphenols From Grape and Blueberry Improve Episodic Memory in Healthy Elderly with Lower Level of Memory Performance: A Bicentric Double-Blind, Randomized, Placebo-Controlled Clinical Study", vol. 74, no. 7, 18 June 2019 (2019-06-18), pages 996 - 1007, XP009514545, ISSN: 1758-535X, Retrieved from the Internet <URL:1077952576> DOI: 10.1093/GERONA/GLY166**
- **PIERRE PHILIP ET AL: "Acute Intake of a Grape and Blueberry Polyphenol-Rich Extract Ameliorates Cognitive Performance in Healthy Young Adults During a Sustained Cognitive Effort", ANTIOXIDANTS, vol. 8, no. 12, 17 December 2019 (2019-12-17), pages 650, XP055762424, ISSN: 2076-3921, DOI: 10.3390/antiox8120650**
- **AL-AWWADI NAJIM A ET AL: "Extracts enriched in different polyphenolic families normalize increased cardiac NADPH oxidase expression while having differential effects on insulin resistance, hypertension, and cardiac hypertrophy in high-fructose-fed rats", JOURNAL OF AGRICULTURAL AND FOOD CHEMISTRY,, vol. 53, no. 1, 12 January 2005 (2005-01-12), pages 151 - 157, XP009173674, ISSN: 0021-8561, DOI: 10.1021/JF048919F**
- **YUN CHEONG-YONG ET AL: "[alpha]-Viniferin Improves Facial Hyperpigmentation via Accelerating Feedback Termination of cAMP/PKA-Signaled Phosphorylation Circuit in Facultative Melanogenesis", vol. 8, no. 7, 1 January 2018 (2018-01-01), AU, pages 2031 - 2043, XP055797490, ISSN: 1838-7640, Retrieved from the Internet <URL:https://www.ncbi.nlm.nih.gov/pmc/articles/PMC5858515/pdf/thnov08p2031.pdf> DOI: 10.7150/thno.24385**
- **ZOLGHADRI SAMANEH ET AL: "A comprehensive review on tyrosinase inhibitors", vol. 34, no. 1, 1 January 2019 (2019-01-01), GB, pages 279 - 309, XP055797497, ISSN: 1475-6366, Retrieved from the Internet <URL:https://www.ncbi.nlm.nih.gov/pmc/articles/PMC6327992/pdf/ienz-34-1545767.pdf> DOI: 10.1080/14756366.2018.1545767**
- **KAZUOMI SATO ET AL: "Depigmenting Effect of Catechins", MOLECULES, vol. 14, no. 11, 4 November 2009 (2009-11-04), pages 4425 - 4432, XP055629257, DOI: 10.3390/molecules14114425**
- **LIKHITWITAYAWUID ET AL: "Stilbenes with tyrosinase inhibitory activity", vol. 94, no. 1, 10 January 2008 (2008-01-10), pages 44 - 52, XP009527078, ISSN: 0011-3891, Retrieved from the Internet <URL:https://wwwops.currentscience.ac.in/Downloads/article_id_094_01_0044_0052_0.pdf>**

(52) Classification Coopérative des Brevets (CPC): (Cont.)

C-Sets
A61K 31/05, A61K 2300/00;
A61K 31/353, A61K 2300/00

## Description

### Domaine technique

L'invention concerne une composition comprenant l'association de monomères de flavanols et d'ε-viniférine comme produit nutritionnel ou médicament destiné à l'Homme ou l'animal.

### Etat de l'art

L'endothélium est une couche monocellulaire tapissant l'intérieur des vaisseaux sanguins jouant le rôle de barrière physique entre la couche tissulaire et la phase circulante. Il permet notamment de réguler le tonus vasculaire. Un dysfonctionnement de la fonction endothéliale ou dysfonction endothéliale est caractérisé par une diminution de la capacité des vaisseaux à se dilater en réponse à une augmentation du flux sanguin. Elle résulte principalement d'une diminution de la disponibilité du monoxyde d'azote (NO), qui peut être due, soit à une réduction de la production de NO, soit à sa captation accrue notamment à cause du phénomène de stress oxydant.

La fonction endothéliale est évaluée par la mesure de la vasodilatation médiée par le flux (souvent désignée par « FMD », acronyme du terme anglais « flow-mediated dilation » ou « dilatation médiée par le flux » en français).

L'endothélium a donc un rôle central dans le maintien du flux sanguin (tant au niveau macro- que microvasculaire). Par conséquent, l'altération de la fonction endothéliale entraîne majoritairement des maladies ou troubles cardiovasculaires, en particulier l'athérosclérose mais également des maladies vasculaires, oculaires, des maladies associées à un déclin cognitif, des troubles fonctionnels intestinaux, des troubles de l'érection, ou des troubles associés à la ménopause.

La dysfonction endothéliale est connue pour être un marqueur prédictif précoce des maladies cardiovasculaires, qui précède ses manifestations cliniques. Or, les maladies cardiovasculaires constituent la première cause de mortalité dans le monde (30% des décès dans le monde) et représentent un problème de santé publique majeur.

Il existe donc un besoin d'identifier un produit capable d'améliorer la fonction endothéliale et permettant de contribuer notamment à la prévention des maladies cardiovasculaires.

Par ailleurs, la dysfonction endothéliale a également été décrite comme un marqueur prédictif du déclin cognitif, y compris en l'absence de maladie cérébro-vasculaire (Naiberg al. Psychosom Med. 2016;78(2):192-207). En effet, le flux sanguin cérébral, qui joue un rôle primordial dans les fonctions cognitives, est étroitement lié à la fonction endothéliale (Fouda et al. Arterioscler Thromb Vasc Biol. 2019 Apr;39(4):593-602).

Préserver une bonne santé vasculaire (et donc une fonction endothéliale optimale) est également important pour la vision. En effet, les maladies cardiovasculaires (diabète, hypertension mais aussi hypercholestérolémie) peuvent engendrer différentes maladies oculaires telles que la rétinopathie hypertensive, la rétinopathie diabétique, le glaucome, etc (Singh et al. Eur J Ophthalmol. 2020 Apr 27:1120672120914232).

Le dysfonctionnement endothélial est également considéré comme l'un des facteurs étiologiques des maladies inflammatoires de l'intestin (Cibor et al. World J Gastroenterol. 2016 Jan 21; 22(3): 1067-1077).

D'autre part, le NO, vasodilatateur, joue également un rôle essentiel dans le déclenchement de l'érection. Ainsi, chez l'homme la dysfonction endothéliale est souvent associée à la dysfonction de la fonction érectile (Kovâcs I et al. J.Cardiovasc Pharmacol, 2008 Feb;51(2):148-53).

Chez la femme, les œstrogènes stimulent la production de NO par les cellules endothéliales. Or, lors de la ménopause, la chute oestrogénique entraîne une forte diminution de la FMD, traduisant une dysfonction endothéliale (Somani YB et al. Am J Physiol Heart Circ Physiol, 2019 Aug 1;317(2):H395-H404). De plus, il a été démontré que le stress mental induit une altération de la fonction endothéliale (Takase B et al. Clin Cardiol. 2004 Apr;27(4):223-7). De la même façon, un déficit de sommeil et certains troubles du sommeil peuvent altérer la fonction endothéliale (Budhiraja et al. J Clin Sleep Med. 2007 Jun 15;3(4):409-15). La fonction endothéliale, tout comme la microcirculation, est également affectée par l'exposition aux UV (Wolf et al. Exp Physiol. 2019;104(7):1136-1146).

Ainsi, la dysfonction endothéliale est la cause ou est impliquée dans de nombreuses pathologies.

Il existe donc un besoin pour maintenir ou améliorer la fonction endothéliale.

Plusieurs études cliniques ont mis en évidence une amélioration significative de la FMD après la consommation d'aliments ou de boissons riches en flavonoïdes. Parmi les flavonoïdes, il a été démontré que la consommation d'une dose unique d'épicatéchine pure permettait d'induire une augmentation significative de la FMD dans un délai de 2 heures (Shafabakhsh et al., Crit Rev Food Sci Nutr. 2020;60(14):2369-2378).

D'autre part, des études épidémiologiques ont révélé une corrélation inverse entre les apports alimentaires en flavonoïdes et le déclin cognitif lié à l'âge. En particulier, il a été démontré que les monomères de flavanols (catéchines et épicatéchines) exercent des effets favorables sur les fonctions cognitives, et que ces effets sont essentiellement dus à la capacité des monomères de flavanols à augmenter le flux sanguin cérébral (Haskell-Ramsay et al., Nutrients. 2018 Jul 27;10(8):986). Les études ayant évalué les effets de l'épicatéchine sur les fonctions cognitives ont mis en évidence des effets favorables lorsque la dose d'épicatéchine était de plus 50 mg/ jour, pendant au moins 28 jours, chez les sujets âgés de plus de 50 ans (Haskell-Ramsay et al., Nutrients. 2018 Jul 27;10(8):986).

Il a également été démontré une augmentation significative de la FMD (et donc une amélioration de la fonction endothéliale), une amélioration du flux sanguin cérébral et certaines performances cognitives après supplémentation en resvératrol (Cicero et al. Arch Med Sci. 2019;15(4):936-943). Des expérimentations précliniques suggèrent que des dimères du resvératrol, en particulier l'ε-viniférine et δ-viniférine, seraient capables d'induire les mêmes effets (Wu et al. The Kaohsiung Journal of Medical Sciences 36 (2020): 535 - 542).

Ainsi, il a déjà été rapporté des effets bénéfiques des flavonoïdes, du resvératrol ou de ses dimères individuellement, sur la fonction endothéliale.

Enfin, l'art antérieur nous enseigne également que Vitis vinifera est une source de monomères de flavanols et d'ε-viniférine (Jayaprakasha et al: "Antioxidant activity of grape seed (Vitis vinifera) extracts on peroxidation models in vitro", 1 mai 2001 ; Gyongyi Nemeth et al: "Stilbenes in the different organs of Vitis vinifera CV. Merlot grafted on TKSBB rootstock", 26 septembre 2017 ; US 2018/271928 ; Al-Awwadi Najim a et al: "Extracts enriched in different polyphenolic families normalize increased cardiac NADPH oxidase expression while having differential effects on insulin resistance, hypertension, and cardiac hypertrophy in high-fructose-fed rats" 12 janvier 2005 ; FR 3 042 712).

Des utilisations associées à ces molécules seules ou en combinaison sont également décrite, tel que dans la mémoire (Bensalem Julien et al: "Polyphenols From Grape and Blueberry Improve Episodic Memory in Healthy Elderly with Lower Level of Memory Performance: A Bicentric Double-Blind, Randomized, Placebo-Controlled Clinical Study" 18 juin 2019), les troubles cognitifs (Pierre Philip et al: "Acute Intake of a Grape and Blueberry Polyphenol-Rich Extract Ameliorates Cognitive Performance in Healthy Young Adults During a Sustained Cognitive Effort, 17 décembre 2019), l'hyperpigmentation (Yun Cheong-Yong et al: "[alpha]-Viniferin Improves Facial Hyperpigmentation via Accelerating Feedback Termination of cAMP/PKA-Signaled Phosphorylation Circuit in Facultative Melanogenesis" 1 janvier 2018, Kazuomi Sato et al: of Catechins" 4 novembre 2009), et comme inhibiteur de la tyrosinase (Zolghadri samaneh et al: "A comprehensive review on tyrosinase inhibitors",1 janvier 2019, Likhitwitayawuid et al: "Stilbenes with tyrosinase inhibitory activity",10 janvier 2008).

Cependant, il existe toujours un besoin important pour un produit capable de prévenir ou traiter la dysfonction endothéliale et maladies associées, les troubles du sommeil, le stress, la cognition ou encore la protection solaire avec une efficacité améliorée par rapport à l'art antérieur.

## Résumé de l'invention

Aussi, l'objectif de l'invention est de fournir une nouvelle composition présentant une efficacité plus importante que les produits existants dans la lutte contre les maladies associées à une dysfonction endothéliale par rapport auxdites molécules spécifiques consommées individuellement.

Les inventeurs ont de façon surprenante identifié une composition comprenant une combinaison spécifique de molécules d'origine naturelle ainsi qu'une quantité spécifique desdites molécules présentes dans ladite composition, présentant un effet synergique et donc une efficacité améliorée de la fonction endothéliale. Ils ont également démontré l'intérêt de ladite composition pour ses effets antioxydants, ainsi que pour protéger la peau des UVB (ultra-violetB), prévenir les érythèmes solaires, prévenir l'hyperpigmentation, prévenir et/ou réduire les taches brunes liées à l'âge. Enfin, la composition est également d'intérêt pour améliorer la mémoire et/ou l'attention et/ou la concentration et/ou la vivacité et/ou la vigilance et/ou l'apprentissage et/ou le langage et/ou l'humeur et/ou le stress et/ou l'anxiété et/ou le sommeil.

En particulier, l'invention a pour objet une composition comprenant spécifiquement au moins une molécule appartenant à la famille des flavonoïdes et au moins une molécule appartenant à la famille des stilbènes. Une telle composition est destinée à être utilisée comme produit nutritionnel, complément alimentaire ou médicament chez l'Homme ou l'animal.

L'invention concerne spécifiquement une composition comprenant un mélange de molécules comprenant :

- au moins 15% de monomères de flavanols, le pourcentage étant donné en poids sec par rapport au poids sec total de la composition, et
- au moins 15 ppm d'epsilon-viniférine (ε-viniférine), en poids sec par rapport au poids sec total de la composition,

ledit mélange de molécules est obtenu à partir d'au moins un extrait ou d'un mélange d'extraits de *Vitis vinifera,* le ratio monomères de flavanols et ε-viniférine est compris entre 20 000 et 4.

Une telle composition présente ainsi un effet synergique en comparaison desdites molécules administrées isolément.

Ainsi, la quantité de monomères de flavanols est supérieure à la quantité d'ε-viniférine, à savoir, le ratio monomères de flavanols et ε-viniférine est compris entre 20000 et 4.

Les monomères de flavanols et l'ε-viniférine sont issus d'un extrait végétal, notamment d'au moins un extrait de *Vitis vinifera* (raisin) et/ou d'un mélange d'au moins deux extraits de *Vitis vinifera*.

La composition peut être administrée par voie orale comme produit nutritionnel, complément alimentaire ou médicament. La composition et/ou une ou plusieurs molécules de la composition peu(ven)t être encapsulée(s) ou microencapsulée(s) dans un support alimentaire. La composition peut se présenter sous forme de poudre, de gélule, de comprimé, de capsule, d'une solution, d'une suspension, d'une émulsion ou d'une gomme à mâcher.

Lorsque la composition est destinée à être utilisée comme médicament ou complément alimentaire, la composition est préférentiellement utilisée pour prévenir et/ou traiter les maladies choisies parmi les maladies cardiovasculaires, les maladies vasculaires, les maladies associées au déclin cognitif, les maladies associées à une perte de la mémoire, les maladies neurodégénératives, les maladies digestives, les maladies articulaires, les maladie érectiles et les troubles associés à la ménopause.

Un autre aspect de l'invention concerne une utilisation non thérapeutique de la composition selon l'invention chez l'Homme ou l'animal sain pour améliorer les fonctions cognitives et/ou les fonctions exécutives, et/ou pour limiter le déclin cognitif normal lié à l'âge et/ou pour améliorer la mémoire et/ou l'attention et/ou la concentration et/ou la vivacité et/ou la vigilance et/ou l'apprentissage et/ou le langage et/ou l'humeur et/ou le stress et/ou l'anxiété et/ou le sommeil et/ou homogénéiser la pigmentation de la peau, et/ou réduire l'apparition de tâches cutanées liées à l'âge et/ou réguler la mélanogénèse et/ou prévenir l'hyperpigmentation de la peau, et/ou prévenir l'érythème solaire, et/ou améliorer l'éclat du teint.

D'autres caractéristiques et avantages ressortiront de la description détaillée de l'invention, des exemples et des figures qui vont suivre.

**Brève description des Figures**

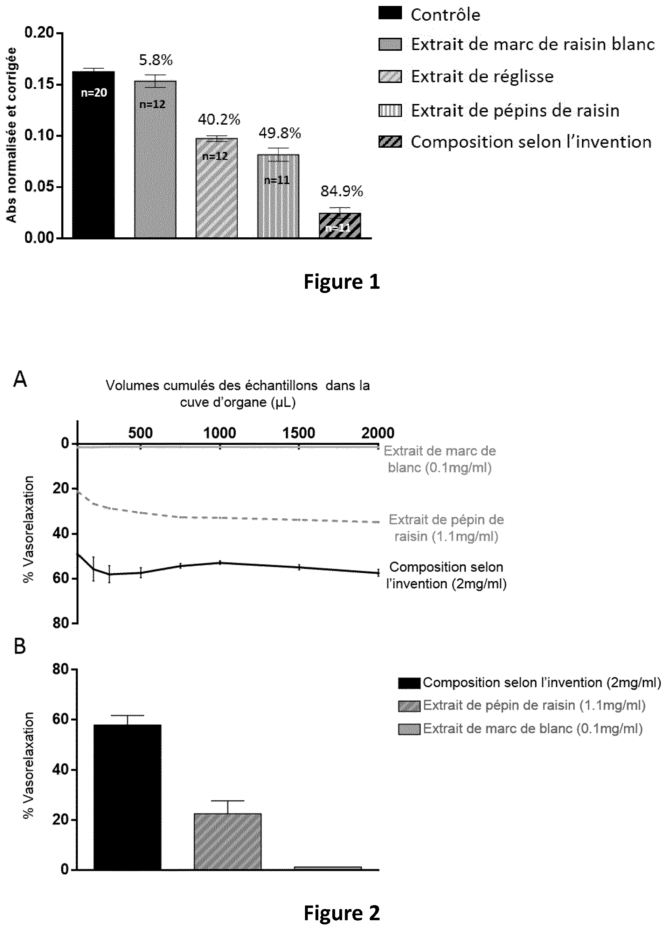

[Fig. 1] La Figure 1 représente, lors de l'évaluation de l'activité de la tyrosinase, la mesure de l'absorbance (normalisées et corrigées) (moyenne $\pm$SEM) à 7min en fonction des différentes compositions testées ainsi que leurs pourcentages d'inhibition respectifs par rapport au contrôle. n : nombre de réplicat. Concentrations testées : l'extrait de marc de raisin rouge : 0.025mg/ml, l'extrait de réglisse : 0,0375mg/ml, l'extrait de pépins de raisin : 0,0286mg/ml, un mélange comprenant une composition selon l'invention : 0.1mg/ml. Le mélange était composé de 58.8% d'une composition selon l'invention composée elle-même de 53.4% de E1 et 46.6% de E3 (la composition selon l'invention apportant 19.4% de monomères de flavanols et 28ppm de ε-viniférine) et de 41.2% d'un extrait de réglisse apportant 8.1% d'acide glycyrrhizique.

Valeur de p pour l'extrait de marc de raisin rouge : <0.724, Valeur de p pour l'extrait de réglisse : <0.0001, Valeur de p pour l'extrait de pépins de raisin :<0.0001, Valeur de p pour l'interaction des 3 extraits :<0.0001.

[Fig. 2] La Figure 2 est A : une représentation des % de vasorelaxation observés (moyenne $\pm$ SEM) ou estimés en fonction des volumes cumulés d'échantillon ajoutés dans la cuve d'organe. Les courbes représentées montrent l'effet d'un mélange testé à 2mg/ml, l'effet estimé d'un extrait de pépin (E1) à 1.1mg/ml, et l'effet estimé d'un extrait de marc de raisin blanc (E5) à 0.1mg/ml. Le mélange testé comprend, quant à lui, 65% d'une composition selon l'invention, composée elle-même de 92.3% de E1 et 7.7% de E5 (la composition selon l'invention apportant 33% de monomères de flavanols et 459ppm d'ε-viniférine) et 35% de maltodextrine. L'effet estimé d'un extrait de pépins à 1.1mg/ml a été comparé préalablement à des données observées. L'analyse statistique par un t-test n'a pas montré de différence significative entre les valeurs estimées et les valeurs observées pour des volumes cumulés compris entre 100 $\mu$l et 2000$\mu$l. B : un histogramme représentant le % de vasorelaxation (moyenne $\pm$ SEM) pour 300$\mu$l d'échantillons ajouté dans la cuve d'organe.

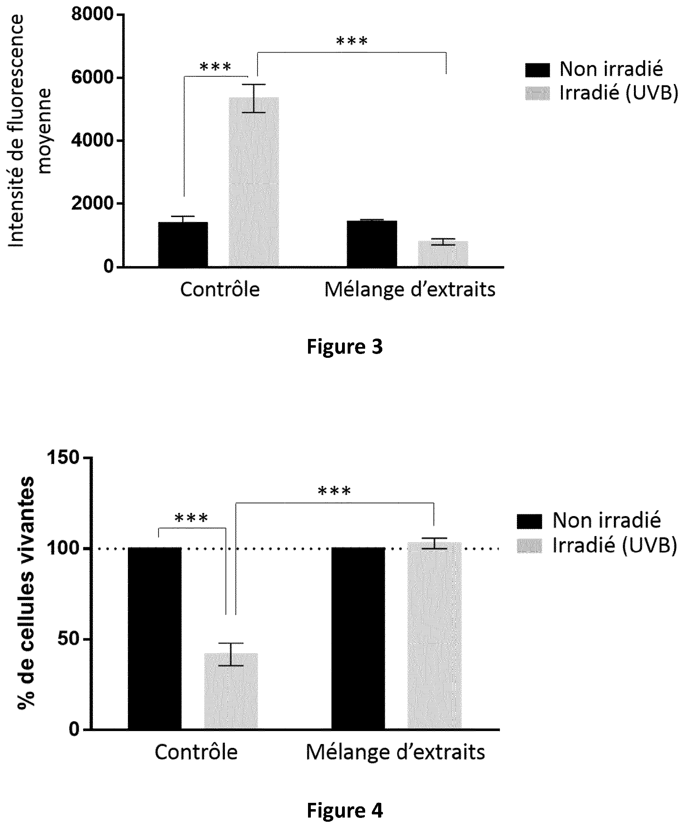

[Fig. 3] La Figure 3 représente l'intensité moyenne de fluorescence (MFI) (moyenne $\pm$ SEM) émise par les cellules marquées au CM-H2DCFDA. Mélange d'extraits : mélange testé à 270.9mg/ml et comprenant : 53.7% d'une composition selon l'invention composée elle-même de 53.4% de E1, et 46.6% de E3 (la composition selon l'invention apportant 19.4% de monomère de flavanols et 28ppm de ε-viniférine) et 37.5% d'un extrait de réglisse. ***p<0.001.

[Fig. 4] La Figure 4 représente le taux de prolifération des kératinocytes exprimé en % (ramené au contrôle non irradié pour chaque condition, moyenne $\pm$ SEM). Mélange d'extraits : mélange testé à 270.9mg/ml et comprenant : 53.7% d'une composition selon l'invention composée elle-même de 53.4% de E1, et 46.6% de E3 (la composition selon l'invention apportant 19.4% de monomères de flavanols et 28ppm de ε-viniférine) et 37.5% d'un extrait de réglisse. ***p<0.001.

**Description détaillée de l'invention**

**Définition**

Par « animal » au sens de l'invention on entend toute animal à l'exclusion de l'Homme.

Par « extrait de *Vitis vinifera* » ou « extrait de raisin » au sens de l'invention on entend un extrait comprenant au moins une molécule, en particulier au moins des monomères de flavanols et/ou l'ε-viniférine, préférentiellement un ensemble de molécules, obtenue(s) à partir de *Vitis vinifera* et/ou d'un sous-produit de *Vitis vinifera*. La matière première peut-être les feuilles et/ou les fruits et/ou les pépins et/ou les pellicules et/ou les rafles et/ou les parties bois (sarments, ceps, racines), et/ou le marc et/ou le vin préférentiellement la matière première est le marc de raisin (comprenant les pépins, les pellicules et éventuellement les rafles) et/ou le pépin de raisin.

Par « ppm » on entend parties par million dans le mélange.

Par « produit nutritionnel » au sens de l'invention on entend un ingrédient alimentaire à vocation nutritionnelle utilisé seul ou associé à d'autres ingrédients ou additifs alimentaires dans des formules alimentaires y compris les compléments alimentaires et aliments destinés à l'Homme ou à l'animal.

Par « médicament » au sens de l'invention on entend un actif employé à des fins thérapeutiques utilisé seul ou associé à d'autres substances actives ou non dans des formules médicamenteuses y compris de phytothérapie ou des compléments alimentaires à effet thérapeutique, destiné à l'Homme ou à l'animal.

Par « prévenir » ou « prévention » au sens de l'invention, on entend la réduction à un degré moindre du risque ou de la probabilité d'occurrence d'un phénomène donné, c'est-à-dire, dans le contexte de la présente invention d'une maladie ou d'un trouble.

Par « traiter » ou « traitement » au sens de l'invention, on entend une diminution de la progression de la maladie ou du trouble, une stabilisation, une inversion ou régression, voire une interruption ou inhibition de la progression d'une maladie ou d'un trouble. Dans le contexte de l'invention, ces termes s'appliquent également sur un ou plusieurs symptômes desdites maladies ou troubles de la présente invention.

**Composition**

La présente invention a donc pour objet une nouvelle composition présentant notamment une efficacité accrue dans l'amélioration ou le maintien de la dysfonction endothéliale et dans la protection solaire.

A cet effet, l'invention vise une composition comprenant un mélange de molécules comprenant :

- au moins 15% de monomères de flavanols, le pourcentage étant donné en poids sec par rapport au poids sec total de la composition, et
- au moins 15 ppm d'ε-viniférine, en poids sec par rapport au poids sec total de la composition,

ledit mélange de molécules est obtenu à partir d'au moins un extrait ou d'un mélange d'extraits de *Vitis vinifera,* le ratio monomères de flavanols et ε-viniférine est compris entre 20 000 et 4.

Les monomères de flavanols sont des composés de la famille des flavonoïdes, en particulier de la sous classe des flavanols comprenant notamment les flavan-3-ols ou flavanols ou catéchines. La classe la plus abondante étant constituée par les catéchines et/ou épicatéchines. Ce sont de puissants antioxydants qui aident notamment à prévenir les maladies inflammatoires et coronariennes mais également à maintenir ou augmenter les performances cognitives. Dans le contexte de l'invention, les monomères de flavanols sont choisis parmi les catéchines, épicatéchines, ainsi que leurs formes galloylés, telles que l'épicatéchine-3-O-gallate, l'épigallocatéchine, l'épicatéchine gallate, l'épigallocatéchine gallate. Ces monomères peuvent être extraits d'un ou plusieurs végétaux (tout ou partie) telles que le thé (Camelia sinensis), les pommes (Malus domestica), le cacao (Theobroma cacao) ou issus de végétaux (tout ou partie) appartenant au genre Vitis et préférentiellement *Vitis vinifera.* Ils peuvent également être issus de microalgues.

L' ε-viniférine est un phénol naturel de la famille des stilbènes, en particulier des stilbénoïdes. L'ε-viniférine est également un dimère du resvératrol. Il peut être extrait d'un ou plusieurs végétaux tels que les végétaux (tout ou partie) appartenant au genre Iris - Iridaceae; Sophora - Fabaceae; Gnetum - Gnetaceae; Carex - Cyperaceae; Pivoine (Paeonia) - Paeoniaceae; Dipterocarpus - Dipterocarpaceae et plus particulièrement appartenant au genre Vitis, préférentiellement *Vitis vinifera*. L'ε-viniférine peut également être issue de microalgues.

Selon un objet de l'invention, la composition comprend préférentiellement au moins 100 ppm de stilbènes comprenant lesdits au moins 15 ppm d'ε-viniférine. Ainsi, la composition selon l'invention comprend au moins 100 ppm de stilbènes dont au moins 15 ppm d'ε-viniférine. En d'autres termes, la composition peut comprendre par exemple 85 ppm de stilbènes autre que l'ε-viniférine et 15 ppm d'ε-viniférine. Lesdits autres stilbènes peuvent être le pinosylvine, le piceatannol, le trans-resvératrol, le trans-ptérostilbène, le rhapontigénine, l'isorhapontigénine, le rhapontine la ponticine, le trans-picéide, ou l'astringine. (delta et/ou omega et/ou alpha et/ou R et/ou R2) viniferine, ampelosine A et/ouE et/ou F et/ou H, miyabenol C, parthenocissine A, pallidol, vitisine C, hopeaphenol, isohopeaphenol, viniferol E.

Selon un mode de réalisation préféré, la composition selon l'invention peut comprendre au moins 20% de monomères de flavanols, le pourcentage étant donné en poids sec par rapport au poids sec total de la composition, plus préférentiellement au moins 25% de monomères de flavanols.

Selon un autre mode de réalisation, la composition selon l'un des quelconques modes de réalisation précédents peut comprendre au moins 50 ppm d'ε-viniférine, en poids sec par rapport au poids sec total de la composition, plus préférentiellement au moins 100 ppm d'ε-viniférine.

Les inventeurs ont identifié que la composition selon l'invention présente une activité améliorée lorsque la quantité en poids de monomères de flavanols est supérieure à la quantité en poids de stilbène, préférentiellement d'ε-viniférine.

Le ratio monomères de flavanols et ε-viniférine est ainsi compris entre 20 000 et 4. Par ratio, on entend le rapport de grandeur entre la quantité de flavanols et la quantité d'ε-viniférine (monomères de flavanols/ε-viniférine). En particulier, le ratio est compris entre 15 000 et 30, encore plus préférentiellement compris entre 10000 et 100.

Les monomères de flavanols sont d'origine naturelle. Ces molécules peuvent ainsi être obtenues à partir d'un produit naturel, à savoir d'un extrait végétal choisi parmi un extrait de *Vitis,* préférentiellement *Vitis vinifera,* un extrait de thé *(Camelia sinensis),* un extrait de pommes (*Malus domestica*), un extrait de cacao (Theobroma cacao). Elles peuvent également être issues de microalgues.

L'ε-viniférine est d'origine naturelle et peut ainsi être obtenue à partir d'un produit naturel, à savoir d'un extrait végétal choisi parmi un extrait de Vitis, préférentiellement *Vitis vinifera.*

Lorsque que les monomères de flavanols et l'ε-viniférine sont obtenus à partir d'un même végétal, le végétal est un extrait de Vitis, notamment un extrait de *Vitis vinifera.*

Il est déjà connu que le raisin et le vin comprennent des monomères de flavanols. Toutefois, la présence de stilbènes et en particulier d'ε-viniférine n'est pas systématique, comme indiqué dans le tableau 1 ci-après. En outre, la concentration en monomères de *Vitis vinifera* ne dépasse jamais naturellement les 10% des polyphénols totaux.

Or, la composition selon l'invention vise un mélange de molécules comprenant au moins 15% de monomères de flavanols. Ainsi, l'origine et le type de la matière première permettant l'obtention du ou des extraits ainsi que le procédé appliqué permettant de l'/les obtenir sont combinés afin d'obtenir un mélange de molécule comprenant au moins 15% de monomères de flavanols et au moins 15 ppm d'ε-viniférine. Les concentrations en molécules en fonction de différentes matières premières de raisin sont donnés dans le tableau 1 ci-après.

[Tableau 1]

| *Vitis vinifera* | Polyphénols Totaux (PT) (Folin) | Monomères de flavanols | | | | stilbènes | |
| | | Catéchine (C) | Epicatéchine (EC) | Total C+EC | [C+EC] /PT | Dont Trans-resvératrol | Dont ε-viniférine |
|---|---|---|---|---|---|---|---|
| Jus de Raisin (mg/100ml )/(%) ou ppm | | 1.21 | 1.26 | 2.47 (0.002%) | | 0.0508ppm | - |
| Raisin noir (mg/100g) /(%) ou ppm | 184.97 (0.18%) | 5.46 | 5.24 | 10.7 (0.01%) | 5.8% | 1.5ppm | - |
| Raisin blanc (mg/100g) /(%) ou ppm | 121.80 (0.12%) | 1.41 | 0.49 | 1.90 (0.002%) | 1.6% | 0.3ppm | - |
| Vin rouge (mg/100m) (%) ou ppm | 215.48 (0.22%) | 6.81 | 3.78 | 10.59 (0.01%) | 4.9% | 1.8ppm | 1.5ppm |
| Vin rosé (mg/100m) /(%) ou ppm | 82.21 (0.08%) | 0.91 | 0.55 | 1.46 (0.001%) | 1.8% | 0.6ppm | - |
| Vin blanc (mg/100ml )/(%) ou ppm | 32.10 (0.03%) | 1.08 | 0.95 | 2.03 (0.002%) | 6.3% | 0.3ppm | 0.0557pp m |
| **Invention** | **>50 %** | | | **>15%** | **>30%** | | **15ppm** |

Selon un mode de réalisation préféré, le ou les extrait(s) de *Vitis vinifera* dans la composition selon l'invention lorsqu'elle on contient, compren(nent) au moins 50% de polyphénols totaux, le pourcentage étant donné en poids sec par rapport au poids total de l'extrait de *Vitis vinifera.*

Préférentiellement, l'extrait de *Vitis vinifera* est un extrait de marc de raisin et/ou de pépin de raisin, plus préférentiellement la composition comprend au moins un extrait de pépin de raisin et/ou un extrait de marc de raisin. Selon un mode de réalisation, la composition selon l'invention comprend au moins un extrait de pépin de raisin et un extrait de marc de raisin.

En plus des monomères de flavanols et de l'ε-viniférine, la composition selon l'invention peut comprendre des dimères (PAC B1 et B2). Préférentiellement la concentration en dimères (PAC B1 et B2) de flavanol est supérieure à 5%, le pourcentage étant donné en poids sec par rapport au poids sec total de la composition.

Lorsque la composition selon l'invention comprend au moins un extrait de marc de raison, préférentiellement le ou les extraits de marc de raisin comprennent préférentiellement des flavonols. De façon préférée, la concentration en flavonols dans la composition est supérieure ou égale à 0,05% en poids sec par rapport au poids sec total de la composition.

La composition selon l'invention peut comprendre en plus du mélange de molécules décrit précédemment au moins un autre constituant choisi parmi les antioxydants, les extraits naturels, les vitamines, les oligo-éléments, les caroténoïdes, les acides gras oméga 3, les huiles naturelles, et leurs mélanges.

Lorsque la composition selon l'invention comprend en outre un extrait naturel autre que l'extrait de *Vitis vinifera,* l'extrait naturel est préférentiellement un extrait de réglisse.

La réglisse est notamment utilisée dans les médecines traditionnelles. Les premières traces de son utilisation documentée en médecine remontent aux anciennes cultures assyrienne, égyptienne, chinoise et indienne.

Les bénéfices qui lui sont attribués sont multiples. En prise orale, la réglisse entrait ainsi dans les remèdes pour les troubles des systèmes respiratoire, gastro-intestinal, cardiovasculaire et génito-urinaire. En topique, elle était utilisée pour le traitement de certaines maladies oculaires ou de la peau (Fiore et al. J Ethnopharmacol 2005 Jul 14;99(3):317-24). Un usage externe, notamment topique est également documenté. Ainsi, l'extrait de réglisse est souvent assimilé dans des formulations cosmétiques visant à améliorer l'aspect de la peau.

Préférentiellement, l'extrait de réglisse comprend de l'acide glycyrrhizique et/ou de la glabridin.

A titre d'exemple d'extraits végétaux supplémentaires, la composition comprend préférentiellement également au moins un extrait choisi parmi les extraits de café, feuilles d'olivier, de cassis, de polygonum, d'agrumes, d'œillet d'Inde et leurs mélanges.

Le ou les polyphénols éventuellement présents dans la composition selon l'invention peuvent être choisis par exemple parmi les polyphénols, notamment sous la forme d'extrait végétaux (extraits de feuilles d'olivier ou d'olives, de cassis, de café, de polygonum, d'agrumes, de câpres), encore la vitamine C, notamment sous la forme d'extraits végétaux (extrait d'acérola, de grenade, d'agrumes), la vitamine E, la vitamine A, notamment sous forme d'extraits végétaux ; ou leurs dérivés, le zinc, le sélénium et leurs mélanges.

La ou les vitamines éventuellement présentes dans la composition selon l'invention peuvent être choisies par exemple parmi la vitamine B9, B12, C, D, E, A dont les pro-vitamines A, leurs dérivés et leurs mélanges.

Le ou les oligo-éléments éventuellement présents dans la composition selon l'invention peuvent être choisis par exemple parmi le zinc, le sélénium ou le chrome et leurs mélanges.

Le zinc est un minéral essentiel au bon fonctionnement cérébral en améliorant la sensibilité à l'insuline, en diminuant le stress oxydant et l'inflammation. D'ailleurs l'agence européenne de sécurité des aliments, dans le cadre du règlement 1924/2006 reconnait le rôle du zinc dans le fonctionnement cognitif normal.

Le ou les caroténoïdes éventuellement présents dans la composition selon l'invention peuvent être choisis par exemple parmi la lutéine, la zéaxanthine, la crocine, la picrocrocine et leurs mélanges.

Le ou les acides-gras moéga-3 éventuellement présents dans la composition selon l'invention peuvent être choisis par exemple parmi l'acide docosahexaénoïque (DHA) et l'acide eicosapentaénoïque (EPA) et leurs mélanges.

Les acides gras oméga 3 à longue chaîne tels que l'acide docosahexaénoïque (DHA) ont un effet bénéfique sur les fonctions cognitives au niveau préclinique chez la souris âgée. Ses effets bénéfiques sur la neuroinflammation sont bien admis aujourd'hui et sont largement décrits dans la littérature.

Les ou les huile(s) naturelle(s) éventuellement présentes dans la composition selon l'invention peuvent être choisis par exemple parmi les huiles de poisson, les huiles végétales les huiles issues de microalgues et leurs mélanges.

La composition peut également comprendre d'autres constituants, comme notamment des excipients, des agents d'enrobage tels que de la maltodextrine, de la cellulose microcristalline, des cyclodextrines, de l'amidon, des fibres solubles ou insolubles, de l'eau ou de l'alcool.

Selon un autre objet de l'invention, la composition et/ou une ou plusieurs molécules de la composition peu(ven)t être encapsulée(s) ou microencapsulée(s) dans un support alimentaire choisi parmi une maltodextrine, une gomme arabique, une huile hydrogénée, une huile non hydrogénée, une cire, un alginate, l'amidon, une protéine et leurs mélanges.

L'encapsulation est particulièrement avantageuse et permet de stabiliser et protéger le mélange de molécules selon l'invention de son environnement, notamment de l'organisme de l'Homme ou l'animal qui ingère la composition. Ainsi, les monomères de flavanols et l'ε-viniférine sont protégés et leur biodisponibilité est améliorée.

De plus, l'encapsulation permet de piéger les métabolites actifs volatils et thermosensibles dans la matrice, d'améliorer la stabilité, la biodisponibilité et l'utilisation dudit mélange de molécules dans les matrices alimentaires, avec notamment un masquage du gout et une résistance aux possibles altérations des composés pendant les étapes de production d'un produit alimentaire.

Selon une variante, la composition et/ou une ou plusieurs molécules peu(ven)t-être microencapsulée(s). La microencapsulation permet de protéger les substances actives au sein de particules de tailles comprises entre 1μm et 1000 μm, plus préférentiellement entre 30 μm et 500μm et encore plus préférentiellement entre 50μm et 300μm.

Selon un autre objet, la composition selon l'invention se présente sous forme de poudre, de gélule, de comprimé, de capsule, d'une solution, d'une suspension, d'une émulsion ou d'une gomme à mâcher.

Lorsque la composition est destinée à être utilisée comme produit nutritionnel, produit alimentaire ou complément alimentaire, la composition peut se présenter sous forme de produit laitier, céréales, produit céréalier ou boisson.

Préférentiellement, la composition selon l'invention est délivrée à une dose permettant de fournir journalièrement à l'Homme ou l'animal au moins 0,25mg par kg de poids corporel de monomères de flavanols et 0,025μg par kg de poids corporel d'ε-viniférine.

**Procédé**

La composition selon l'invention peut être obtenue par tout procédé permettant d'obtenir un mélange de molécules comprenant au moins 15% de monomères de flavanols en poids par rapport au poids total de la composition et d'au moins 15 ppm de d'ε-viniférine en poids par rapport au poids total de la composition. Il peut s'agir du mélange des différentes molécules dans les proportions recherchées ou bien du mélange d'extraits de végétaux comprenant les différentes molécules dans les proportions recherchées.

Selon un mode de réalisation, la composition selon l'invention est obtenue à partir d'un extrait de *Vitis vinifera* ou d'un mélange d'extraits de *Vitis vinifera.* Les extraits peuvent être obtenus par tout procédé permettant d'obtenir un mélange comprenant au moins 15% de monomères de flavanols en poids et d'au moins 15 ppm d'ε-viniférine en poids.

Un procédé particulièrement adapté pour obtenir un extrait de *Vitis vinifera* comprend les étapes suivantes :

- extraction à l'eau et/ou à l'alcool (notamment éthanol) de *Vitis vinifera* (par exemple fruit, pellicule, pépin, feuille, rafle, sarment, ceps, bois, racine), préférentiellement de pépins de *Vitis vinifera* et/ou de marc de *Vitis vinifera* (le marc contenant des pellicules de raisin c'est-à-dire de la peau de raisin, des pépins de raisins et éventuellement des rafles), les pépins et/ou le marc pouvant être issus notamment des résidus de pressage de la fabrication du vin, et/ou de feuilles de *Vitis vinifera* et/ou de sarments, ceps, bois, racines de *Vitis vinifera.*

   La quantité d'eau ou de solution hydroalcoolique (30% v/v à 96% V/V) mise en œuvre est préférentiellement comprise entre 2 et 10 fois la masse de matière mise en œuvre. La durée de l'extraction peut être comprise entre 30 minutes et 24 heures et la température d'extraction comprise entre 20°C et 80°C.
   Les matières premières utilisées peuvent être sous formes sèches, fraîches, ou congelées entières ou broyées ;

- séparation de la solution d'eau et/ou alcool de la matière solide, par exemple par décantation centrifuge ou par pressage et filtration ; l'extrait ainsi obtenu est appelé extrait brut A,
- possiblement pourra être appliqué une élimination de l'alcool par évaporation sous-vide à une température, préférentiellement inférieure à 60°C et à une pression inférieure à 100mbars.

Si on utilise plusieurs matières premières, par exemple des pépins de raisin et du marc de raisins, le procédé peut comprendre une seule extraction à partir des deux matières premières mélangées ou bien le procédé peut consister à obtenir deux extraits séparés (mises en œuvre de tout ou partie du procédé de façon séparée pour chaque extrait, puis mélange à la fin des deux extraits obtenus).

Sur la base du ou des extrait(s) brut(s) A préalablement obtenu(s) le procédé peut comprendre une étape spécifique pour permettre d'obtenir un ou des extrait(s) purifié riche(s) en monomères de flavanols et/ou en-viniferine.

Pour les monomères de flavanols la purification peut être réalisée soit :

- par purification par séparation membranaire d'ultrafiltration et/ou de nanofiltration connue de l'homme de l'art de l'extrait préalablement désolvanté de façon à sélectionner préférentiellement les monomères et d'éliminer les polymères de flavanols, pour obtenir un extrait comprenant une fraction enrichie en monomères de flavanols,
- par purification à l'aide d'une séparation liquide/liquide par des solvants organiques connu de l'homme de l'art et en particulier par de l'acétate d'éthyle,

- par purification via une précipitation aux sels tel le NaCl des polymères de flavanols suivi d'une séparation solide/liquide telles que la filtration par exemple.

L'extrait ainsi obtenu est un extrait purifié riche en monomères de flavanols appelé extrait B.

Le procédé comprend préférentiellement une étape supplémentaire de purification chromatographique de l'extrait A et/ou de l'extrait B. Cette étape consiste à passer sur une colonne chromatographique contenant des résines d'adsorption particulièrement adaptée aux polyphénols et connues de l'homme de l'art, les extraits A et/ou B puis à rincer les résines à l'aide d'une solution aqueuse suivie d'une phase d'élution à l'aide d'une solution hydro éthanolique comprise de préférence entre 60 et 80% v/v.

L'alcool des éluats ainsi obtenus est évaporé et l'éluat concentré via une étape de concentration sous-vide de/des éluats riches en polyphénols et plus particulièrement en monomères de flavanols et/ou en ε-viniférine à une température préférentiellement inférieure à 60°C et une pression inférieure à 100 mbars.

Le procédé peut comprendre une étape supplémentaire qui consiste à sécher les extraits purifiés par des techniques connues de l'homme de l'art telle que l'atomisation, le séchage dans une étuve sous vide ou séchage par lyophilisation avec ou sans support telle que la maltodextrine, la gomme arabique par exemple.

Le procédé peut aussi comprendre une étape d'encapsulation ou de microencapsulation des molécules et/ou de la composition.

Une fois l'extrait obtenu, le procédé de préparation de la composition selon l'invention peut comprendre le mélange d'extraits de *Vitis vinifera* riches d'une part en monomères de flavanols et d'autre part en ε-viniférine.

**Utilisation**

Selon un autre aspect, l'invention concerne également la composition selon l'un des quelconques modes de réalisation précédent pour son utilisation en tant que médicament pour l'Homme ou l'animal.

Préférentiellement, la composition selon l'invention est destinée à être utilisée dans la prévention et/ou le traitement des maladies associées à une dysfonction endothéliale choisies parmi les maladies cardiovasculaires, les maladies vasculaires, les maladies associées au déclin cognitif, les maladies associées à une perte de la mémoire, les maladies neurodégénératives, les maladies digestives, les maladies articulaires, les maladies oculaires, les troubles érectiles et les troubles associés à la ménopause.

Lorsque la composition est destinée à prévenir et/ou traiter les maladies associées au déclin cognitif dont les maladies neurodégénératives ou les maladies associées à une perte de la mémoire, ladite maladie est choisie parmi la maladie d'Alzheimer, la maladie de Parkinson, la maladie de Huntington, les maladies du motoneurone, la démence, la dépression, l'anxiété, la schizophrénie, le retard mental et le syndrome de dysfonctionnement cognitif (CDS).

Lorsque la composition est destinée à prévenir et/ou traiter les maladies cardiovasculaires, ladite maladie est choisie parmi l'hypertension artérielle, l'athérosclérose, l'infarctus du myocarde, l'angine de poitrine (angor)

Lorsque la composition est destinée à prévenir et/ou traiter les maladies vasculaires, ladite maladie est choisie parmi l'insuffisance veineuse chronique, la thrombose veineuse, les varices.

Lorsque la composition est destinée à prévenir et/ou traiter les maladies digestives, ladite maladie est choisie parmi le syndrome de l'intestin irritable, la maladie de Crohn, la colite ulcéreuse et la dyspepsie.

Lorsque la composition est destinée à prévenir et/ou traiter les maladies articulaires, ladite maladie est choisie parmi l'arthrose, l'arthrite rhumatoïde, la polyarthrite rhumatoïde et la spondylarthrite ankylosante.

Lorsque la composition est destinée à prévenir et/ou traiter les maladies oculaires, ladite maladie est choisie parmi la dégénérescence maculaire liée à l'âge (DMLA), la rétinopathie hypertensive, la rétinopathie diabétique, le glaucome, la cataracte ou la dystrophie endothéliale de Fuchs

Lorsque la composition est destinée à prévenir et/ou traiter les troubles du sommeil, ledit trouble est choisi parmi l'insomnie, la parasomnie, l'apnée et/ ou l'hypopnée du sommeil.

Lorsque la composition est destinée à prévenir et/ou traiter les_troubles associés à la ménopause, ledit trouble est choisi parmi les bouffées de chaleur, les sueurs nocturnes, l'augmentation de la masse grasse abdominale, la sècheresse vaginale, la baisse de la libido, les troubles de l'humeur.

Selon un autre aspect, l'invention concerne également une utilisation non thérapeutique de la composition selon l'invention chez l'Homme ou l'animal sain, pour améliorer les fonctions cognitives et/ou les fonctions exécutives, et/ou pour limiter le déclin cognitif normal lié à l'âge et/ou pour améliorer la mémoire et/ou l'attention et/ou la concentration et/ou la vivacité et/ou la vigilance et/ou l'apprentissage et/ou le langage et/ou l'humeur et/ou le stress et/ou l'anxiété et/ou le sommeil et/ou homogénéiser la pigmentation de la peau, et/ou améliorer la beauté de la peau et/ ou réduire l'apparition de tâches cutanées liées à l'âge, et/ou réguler la mélanogénèse et/ou hyperpigmentations dues à une surproduction de mélanine et/ou la prévention des dommages et/ou désordres cutanés liés à l'exposition solaire tel que les érythèmes et le vieillissement cutané.

Lorsque la composition selon l'invention est destinée à être utilisée pour homogénéiser la pigmentation de la peau, et/ou améliorer la beauté de la peau et/ ou réduire l'apparition de tâches cutanées liées à l'âge, et/ou réguler la mélanogénèse, et/ou hyperpigmentations dues à une surproduction de mélanine, et/ou la prévention des dommages et/ou désordres cutanés liés à l'exposition solaire tel que les érythèmes et le vieillissement cutané, la composition comprend préférentiellement au moins un extrait de réglisse.

Par hyperpigmentations dues à une surproduction de mélanine, on entend au sens de l'invention une hyperpigmentation post-inflammatoire, ou due à une phytophotodermatite, à un processus néoplasique (lentiligos, mélanome), aux mélasma, aux taches de rousseur, à l'acanthosis nigricans, résultant de prise de médicaments ou de l'exposition solaire.

L'invention est à présent illustrée par des exemples non limitatifs de compositions selon l'invention, d'extrait selon l'invention, d'extrait hors-invention et par des résultats d'efficacité.

**Exemples**

**Exemple** 1 : **Extrait de pépin de raisin riche en monomères de flavanols.**

L'extrait est obtenu selon le procédé suivant :

- 40 kg de pépins de raisin secs extraction aqueuse dans 400kg d'eau chauffée à 85°C
- Séparation liquide/solide puis l'extrait aqueux est alors passé sur une membrane < 15kD
- Le perméat est alors passé sur une colonne chromatographique d'adsorption C18
- Purification sur résine d'adsorption 10 BV à 2 BV/h,
- Lavage à l'eau 4 BV a 2BV/h
- Elution hydro éthanolique avec solution 80% V/V à 1BV/H avec 2BV
- Concentration sous vide et désalcoolisation sous 60 mbar à 40°C
- Séchage par atomisation

L'extrait de pépin de raisin ainsi obtenu comprend au moins 15% de monomères de flavanol et comprenant au moins 50% de polyphénol totaux, mesuré par la méthode de Folin. Un tel extrait (E1) est caractérisé dans le tableau 2 ci-après.

**Exemple 2 : Extrait de pellicule de raisin rouge riche en anthocyanes mais sans stilbènes.**

L'extrait est obtenu selon le procédé suivant :

- 40kg de marc de raisin rouge
- Extraction avec 200kg Solution aqueuse avec 0.1% de sulfites
- Concentration sous vide 100mbar à 50°C élimination partielle des sulfites
- Séchage par atomisation.

L'extrait de raisin ainsi obtenu (E2) est caractérisé dans le tableau 2 ci-après.

**Exemple 3 : Extrait de marc de raisin comprenant des stilbènes.**

L'extrait est obtenu selon le procédé suivant :

- Extraction hydro alcoolique
- Extrait de marc de raisin rouge (E3)
- 10kg de marc sec extraction hydro alcoolique à 30% V/V ratio solide liquide 1/10 agitation à 350 rpm à 80°C pendant 4 heures
- Séparation solide liquide par filtration à 20$\mu$m
- Concentration sous vide à 40°C
- Séchage par atomisation

L'extrait de raisin ainsi obtenu (E3) est caractérisé dans le tableau 2 ci-après.

**Exemple 4** : **Extrait de marc de raisin rouge purifié comprenant des stilbènes.**

L'extrait est obtenu selon le procédé suivant :

- Extraction 80°C pendant 4H à 50% v/v EtOh ratio 1/10
- 10kg de marc sec extraction hydro alcoolique à 30% V/V ratio solide liquide 1/10 agitation à 350 rpm à 80°C pendant 4 heures
- Séparation solide liquide par filtration à 20μm
- Concentration sous vide à 40°C
- Purification sur résine d'adsorption 20 BV à 2 BV/h
- Lavage à l'eau 4 BV a 2BV/h
- Elution hydro éthanolique avec solution 80% V/V à 1BV/H avec 2BV
- Concentration sous vide et désalcoolisation sous 200 mbar à 40°C
- Séchage par lyophilisation

L'extrait de raisin ainsi obtenu (E4) est caractérisé dans le tableau 2 ci-après.

**Exemple 5** : **Extrait de marc de raisin blanc comprenant des stilbènes.**

L'extrait est obtenu selon le procédé suivant :

- 125kg de marc de raisin blanc extraction hydro alcoolique à 30% V/V ratio solide liquide 1/10 agitation à 350 rpm à 80°C pendant 4 heures
- Séparation solide liquide par filtration à 20μm
- Concentration/désalcoolisation sous vide sous 100 mbar à 40°C
- Purification sur résine d'adsorption 20 BV à 2 BV/h
- Lavage à l'eau 4 BV a 2BV/h
- Elution hydro éthanolique avec solution 80% V/V à 1BV/H avec 2BV
- Concentration sous vide et désalcoolisation sous 200 mbar à 40°C
- Séchage par atomisation.

L'extrait de raisin ainsi obtenu (E5) est caractérisé dans le tableau 2 ci-après.

**Exemple 6 : Extrait de sarment de vigne comprenant des stilbènes mais sans monomères de flavanols.**

L'extrait est obtenu selon le procédé suivant :

- 40g de sarments extraction hydroalcoolique dans 250g d'une solution à 90%V/V à 80°C pendant 4H
- Séparation solide liquide filtration
- Désalcoolisation et Concentration sous vide
- Séchage par lyophilisation.

L'extrait de *Vitis vinifera* obtenu (E6) comprend 5.1% de resvératrol, 8.5% d'ε-viniférine, 15,5% de stilbènes et 0% de catéchine et d'épicatéchine

[Tableau 2]

| Polyphenols par UHPLC-DAD-FLD | Extrait de pépin de raisin (E1) | Extrait de marc de raisin rouge riche en anthocyanes (E2) | Extrait de marc de raisin rouge (E3) | Extrait de marc de raisin rouge purifié (E4) | Extrait de marc de raisin blanc (E5) | Extrait de sarment de vigne (E6) |
|---|---|---|---|---|---|---|
| Gallic acid | **0.6634%** | **0.1109%** | **0.1912%** | **0.0609%** | **0.0294%** | n.a. (<0.0005%) |
| | | | | | | |
| **Flavanols** | | | | | | |
| Catechin | 21.75% | | 0.47% | 5.69% | 1,22% | n.a. (<0.0005%) |
| Epicatechin | 13.84% | | 0.30% | 3.88% | 0,88% | n.a. (<0.0005%) |
| **Total monomers hors epicatechin gallate** | 35.59% | | **0.77%** | **9.57%** | **2,10%** | **n.a. (<0.0005%)** |
| Epicatechin gallate | 2.55% | | 0.17% | 0.7% | 0,53% | n.a. (<0.0005%) |
| **Total monomers** | **38.14%** | | **0.94%** | **10.27%** | **2.63%** | **n.a. (<0.0005%)** |
| PAC B2 (dimer)* | 4.29% | | 0.19% | 0.89% | 0,28% | n.a. (<0.0005%) |
| PAC B1 (dimer)* | 5.06% | | 0.18% | 1.25% | 0,25% | n.a. (<0.0005%) |
| PAC B2-gallate | 3.78% | | 0.09% | 0.43% | 0,45% | n.a. (<0.0005%) |
| **Total PAC dimer** | **13.13%** | | **0.46%** | **2.57%** | **0.98%** | **n.a. (<0.0005%)** |
| **Flavonols total quercetin derivatives** | **n.a. (<0.0005%)** | **0.5249%** | **0.4343%** | **0.6582%** | **0,5829%** | **n.a. (<0.0005%)** |
| Stilbènes | | | | | | |
| trans-Resveratrol | n.a. (<0.0005%) | n.a. (<0.0005%) | 0.0146% | 0.1035% | 0,1146% | 5,34% |
| Piceid | n.a. (<0.0005%) | n.a. (<0.0005%) | n.a. (<0.0005%) | n.a. (<0.0005%) | 0,0343% | 0,0513% |
| Piceatannol | n.a. (<0.0005%) | n.a. (<0.0005%) | n.a. (<0.0005%) | n.a. (<0.0005%) | 0,0589% | 0,1424% |
| e-Viniferin | n.a. (<5ppm) | n.a. (<5ppm) | 61ppm | 529ppm | 5962ppm | 85600ppm |
| R-Viniferin | n.a. (<0.0005%) | n.a. (<0.0005%) | 0.0035% | 0.0159% | 0,0392% | 1,4383% |
| Viniferin 2 | n.a. (<0.0005%) | n.a. (<0.0005%) | n.a. (<0.0005%) | 0.0041% | 0,1130% | 1,2818% |
| Viniferin 3 | n.a. (<0.0005%) | n.a. (<0.0005%) | 0.0200% | n.a. (<0.0005%) | 0,0517% | 0,4347% |
| Viniferin 5 | n.a. | n.a. (<0.0005%) | 0.0135% | n.a. | 0,1110% | 0,4712% |

| | (<0.0005%) | | | (<0.0005%) | | |
|---|---|---|---|---|---|---|
| Total Stilbenoïds | n.a. (<0.0005%) | n.a. (<0.0005%) | 0.0577% | 0.1734% | 1,1189% | 17,7572% |
| Total Polyphénols (Folin) | 99.6% | 31.1% | 36.8% | 57,9% | 55,3% | 53.3% |
| Ratio monomères de flavanols sur Polyphénols totaux | 35.73% | - | 2.1% | 16.5% | 3.8% | 0 |

## Exemple 7 : Exemples de compositions selon l'invention

Le tableau 3 ci-après présente plusieurs exemples de composition comprenant des extraits issus des procédés décrits précédemment dans les exemples 1 à 6.

[Tableau 3]

| Exemples selon l'invention | Formulation | Monomères de flavanols | Dimères (PAC B2 et B1) de flavanols | E-viniferine | Stilbènes | Ratio Monomères /$\varepsilon$-viniferine | Flavonols | P Polyphénols totaux |
|---|---|---|---|---|---|---|---|---|
| A | 75%E1+25%E3 | 26.9% | 10,0% | 0.0015 % | 0.0144% | 17639/1 | 0.11% | 83% |
| B | 75%E1+25%E4 | 29.1% | 10.6% | 0.0132 % | 0.0434% | 2200/1 | 0.16% | 74% |
| C | 99%E1+1%E5 | 35.3% | 9.8% | 0.0060 % | 0.0112% | 6272/1 | 0.05% | 99% |
| D | 40%E1+60%E5 | 15.5% | 5.3% | 0.3577 % | 0.6713% | 43/1 | 0.34% | 82% |
| E | 40%E1+35%E2+25%E3 | 15.5% | 5.4% | 0.0015 % | 0.0144% | 10160/1 | 0.11% | 60% |
| F | 50%E1+50%E6 | 17.8% | 6.6% | 4.2800 % | 8.8700% | 4.15/1 | - | 76% |

Il est à noter que la concentration en dimères (PAC B1 et B2) de flavanol est supérieure à 5% en poids sec du poids de la composition. En outre pour les extraits de *Vitis vinifera* issus des parties du marc (pépin, pellicule rafle) la concentration en flavonols est égal ou supérieure à 0.05% en poids sec du poids de la composition.

## Exemple 8 : Evaluation de l'effet de composition selon l'invention sur l'activité de la tyrosinase.

### Evaluation de l'effet d'une composition comprenant des monomères de flavanols et de l'$\varepsilon$-viniférine

L'objectif de cet essai est de modéliser l'activité d'une enzyme, la tyrosinase (enzyme responsable de la formation de mélanine) à l'aide d'un plan expérimental et de déterminer l'influence de monomères de flavanols et de l'$\varepsilon$ viniférine sur l'activité enzymatique vis-à-vis d'un substrat spécifique (L-DOPA). L'activité de la tyrosinase a été mesurée in vitro par une réaction colorimétrique. Sous l'action de la tyrosinase, la L-DOPA est oxydée et est convertie en dopaquinone. La formation de dopaquinone peut alors être suivie en spectrométrie à 475nm.

Dans une plaque 96 puits, les quantités suivantes ont été déposées dans chaque puits : 80$\mu$l tampon phosphate (50mM), 40$\mu$l d'échantillon à tester ou de tampon, 40$\mu$l de tyrosinase à 125U/ml. Au dernier moment, 40$\mu$l de L-DOPA (2.5mM) a été ajouté pour initier la réaction puis un suivie de l'absorbance à 475nm a été réalisé pendant 25min.

Pour chaque puits, les valeurs d'absorbance au cours du temps ($A_x$) sont normalisées par rapport à la première mesure d'absorbance ($A_{TO}$) selon la formule suivante : Absorbance normalisée ($A_n$) = $A_x$ - $A_{TO}$. Les absorbances normalisées sont ensuite corrigées (pour chaque temps) selon la formule suivante : Absorbance normalisée corrigée ($A_{nc}$) = $A_n$ - $A_{CLT}$ où $A_{CLT}$ est la valeur de l'absorbance normalisée des réactifs en présence de l'échantillon (tampon + tyrosinase + échantillon). Plus les valeurs ($A_{nc}$) observées sont basses, plus l'activité de la tyrosinase est diminuée (vis-à-vis du substrat L-DOPA).

Pour réaliser cet essai, un logiciel design expert a été utilisé, et un plan composite centré comme plan expérimental avec pour les facteurs, des concentrations comprises entre 1 et 50 $\mu$M pour les monomères de flavanols et entre 0.0050 et 0.05 $\mu$M pour l'ε-viniférine et pour la réponse, l'absorbance normalisée corrigée mesurée après 7 min. Le ratio monomères de flavanols et ε-viniférine est compris entre 10000 et 1000.

Les résultats obtenus ont montré que l'absorbance normalisée corrigée pouvait être modélisée par un modèle linéaire quadratique ($p < 0.0001$) (Tableau 4).

[Tableau 4]

| Source | Somme des carrées | df | Moyenne quadratique | F-value | p-value |
|---|---|---|---|---|---|
| **Model** | 0.0349 | 5 | 0.0070 | 27.53 | < 0.0001 |
| A-Flavanols | 0.0275 | 1 | 0.0275 | 108.46 | < 0.0001 |
| B-ε-Viniferin | 0.0005 | 1 | 0.0005 | 1.88 | 0.1780 |
| AB | 0.0023 | 1 | 0.0023 | 9.00 | 0.0047 |
| $A^2$ | 0.0030 | 1 | 0.0030 | 11.76 | 0.0015 |
| $B^2$ | 0.0006 | 1 | 0.0006 | 2.42 | 0.1285 |
| Residual | 0.0096 | 38 | 0.0003 | | |
| Lack of Fit | 0.0019 | 3 | 0.0006 | 2.86 | 0.0509 |
| | | | | | |
| | Valeur | | | | |
| $R^2$ | 0.7837 | | | | |
| Adjusted $R^2$ | 0.7552 | | | | |
| Predicted $R^2$ | 0.7025 | | | | |

Les résultats démontrent également un effet significatif des monomères de flavanols sur l'activité de la tyrosinase vis-à-vis du substrat L-DOPA. L'analyse des coefficients en termes de facteurs codés (Tableau 5) montre que les monomères de flavanols diminuent l'activité de la tyrosinase vis-à-vis de son substrat, L-DOPA.

[Tableau 5]

| Facteur | Coefficient Estimé | df | Erreur standard | 95% IC (bas) | 95% IC (haut) |
|---|---|---|---|---|---|
| Intercept | 0.1508 | 1 | 0.0041 | 0.1426 | 0.1591 |
| A-Flavanols | -0.0338 | 1 | 0.0032 | -0.0404 | -0.0273 |
| B-ε-Viniferin | -0.0045 | 1 | 0.0032 | -0.0110 | -0.0021 |
| AB | -0.0119 | 1 | 0.0040 | -0.0200 | -0.0039 |
| $A^2$ | 0.0171 | 1 | 0.0050 | 0.0070 | 0.0273 |
| $B^2$ | 0.0078 | 1 | 0.0050 | -0.0024 | 0.0179 |

En revanche, aucun effet significatif de l'ε-viniférine n'a été observé dans la plage de concentration testées dans le plan expérimental. Enfin, de manière intéressante, une interaction significative entre les monomères de flavanols et l'ε-viniférine a été observée. De plus, le coefficient de l'interaction de ces facteurs étant négatif, cela démontre que l'association de monomères de flavanols et d'ε-viniférine diminue également l'activité de la tyrosinase vis-à-vis du substrat L-DOPA.

L'effet de la combinaison de monomères de flavanols et de l'ε-viniférine est supérieur à un effet additif des molécules. En effet, pour une concentration en monomères de flavanols à 35$\mu$M et une concentration en $\varepsilon$-viniferine à 0.005$\mu$M, une valeur d'absorbance à 0.158 est obtenue alors que la valeur de l'absorbance pour un effet additif est de 0.332. Par conséquent, la composition selon l'invention présente bien un effet synergique.

**Evaluation de l'effet d'un mélange d'extraits de *Vitis vinifera* et d'extrait de réglisse.**

L'objectif de cet essai est d'évaluer l'activité de la tyrosinase (vis-à-vis du substrat L-DOPA) en présence d'un mélange comprenant :

- 58.8% d'une composition selon l'invention composée elle-même de 53.4% de E1 et 46.6% de E3 (la composition selon l'invention apportant 19.4% de monomères de flavanols et 28ppm de $\varepsilon$-viniférine) et
- 41.2% d'un extrait de réglisse apportant 8.1% d'acide glycyrrhizique.

Cette activité sera comparée aux activités enzymatiques en présence d'E1 ou d'E3 ou de l'extrait de réglisse seul.

Pour ce faire l'activité de la tyrosinase a été suivie et mesurée in vitro par la réaction colorimétrique décrite précédemment. Les extraits ont été testés aux concentrations suivantes : extrait de pépins de raisin à 0.028mg/ml, extrait de marc de raisin rouge à 0.025mg/ml, extrait de reglisse à 0.037mg/ml.

Le pourcentage d'inhibition de la tyrosinase a été déterminé sur les absorbances normalisées et corrigée, au T=7min, selon l'équation suivante.

$$\% \text{ d'inhibition} = \frac{A_{nc} \text{ Contrôle} - A_{nc} \text{ Echantillon}}{A_{nc} \text{ Contrôle}} \times 100$$

Où $A_{nc}$ Contrôle est la moyenne des mesures d'absorbance normalisée et corrigés des réaction normales (tampon + tyrosinase + L-DOPA) et $A_{nc}$ Echantillon est l'absorbance normalisée corrigée de la réaction en présence de l'échantillon (tampon + tyrosinase+ L-DOPA+ échantillon).

Les valeurs d'absorbances (à T=7min) ont été comparées statistiquement en utilisant une analyse de variance (ANOVA) à trois facteurs ou le facteur 1 était le traitement avec un extrait de pépins de raisin, le facteur 2 était le traitement avec un extrait de marc de raisin rouge, le facteur 3 était le traitement avec un extrait de réglisse. Une valeur de p<0.05 est considérée comme significative.

En présence d'un extrait de marc de raisin rouge, une faible et non significative diminution de l'activité de la tyrosinase a été observée (Figure 1). En présence d'un extrait de réglisse ou d'un extrait de pépin de raisin, des diminutions significatives, respectivement de 40.2% et de 49.8% de l'activité de la tyrosinase ont été observées. Enfin une interaction significative a été trouvée entre un extrait de pépins, un extrait de marc de raisin rouge et un extrait de réglisse. Ainsi un tel mélange selon l'invention a montré une diminution (84.9%) de l'activité de la tyrosinase plus importante que celle observé pour les extraits de marc de raisin rouge, un extrait de pépins de raisin ou un extrait de réglisse seuls. La diminution de l'activité de la tyrosinase vis-à-vis du substrat L-DOPA pourrait ainsi permettre la diminution de la production de mélanine et par conséquent améliorer l'homogénéité de la peau et réduire les taches de pigmentation liées à l'âge ou des processus d'hyperpigmentation.

**Exemple 9 : Evaluation de l'effet d'une composition selon l'invention sur la fonction endothéliale.**

Cet essai vise à évaluer la vasorelaxation, ex vivo, d'aortes de rats, en présence d'un mélange comprenant :

- 65% d'une composition selon l'invention composée elle-même de 92.3% de E1 et 7.7% de E5 (la composition selon l'invention apportant 33% de monomères de flavanols et 459ppm d'$\varepsilon$-viniférine, et
- 35% de maltodextrine

La vasorelaxation induite par ce mélange sera comparée à la vasoralaxation induite par les extraits E1 et E5 seuls.

Pour ce faire, des aortes thoraciques de rat ont été prélevées, nettoyées et sectionnées en anneaux de 2-3mm. Les anneaux sont ensuite immergés dans une cuve à organes isolés, dans 20ml de solution Krebs (118 mM NaCl, 25 mM NaHCO3, 5.5 mM d-glucose, 4.7 mM KCl, 1.18 mM KH2PO4, 2.4 mM MgSO4, and 3.3 mM CaCl2, pH 7.8) à 40°C et bullée en continu avec 95% O2 et 5% CO2. Pour tester la réactivité de l'endothélium, des contractions ont été induites avec du KCl (80mM) jusqu'à atteindre un plateau. La cuve à organes a ensuite été lavée 3 fois avec de la solution Krebs. L'endothélium fonctionnel a ensuite été testé en ajoutant d'abord 0,5% de noradrénaline (à 0,1 $\mu$M), pour induire la contraction, puis de l'acétylcholine (à 10 $\mu$M), pour induire au moins 80% de vasorelaxation. Des volumes cumulés d'échantillon ont ensuite été ajoutés dans la cuve d'organe pour atteindre un volume final de 2000 $\mu$L sur une période de 1 h et une courbe concentration-relaxation a été construite. Trois tests indépendants ont été effectués pour chaque échantillon.

Les résultats montrent que le mélange comprenant une composition selon l'invention induit une forte vasorelaxation des sections d'aorte (Figure 2A). En effet, la vasorelaxation maximale observée est de 58% et est atteinte après ajout de 300μL d'échantillon. En présence du mélange comprenant une composition selon l'invention, l'effet sur la vasorelaxation est plus important que celui observé pour un extrait de pepin de raisin seul (et à la même concentration que dans le mélange comprenant une composition selon l'invention) et celui estimé pour un extrait de marc de raisin blanc seul (et à la même concentration que dans le mélange comprenant une composition selon l'invention) (Figure 2B). Le mélange comprenant une composition selon l'invention induit également un effet supérieur à l'addition des effets des extraits (hors invention) pris séparément. Ces résultats démontrent qu'il existe des interactions synergiques entre les monomères de flavanols et ε-viniférine dans le mélange comprenant une composition selon l'invention. Un tel effet synergique de la composition de l'invention permet de proposer des formulations améliorant la fonction endothéliale et donc la fonction vasculaire.

## Exemple 10 : Evaluation de la capacité antioxydante d'un mélange d'extraits de *Vitis vinifera*

L'objectif de cet essai est de déterminer l'activité antioxydante d'extraits de *Vitis vinifera* tel que l'extrait de pépin de raisin ou d'un mélange d'extraits de *Vitis vinifera* comprenant 50% de E1 et 50% de E3 en la comparant à un analogue de la vitamine E : le trolox. L'activité antioxydante a été mesurée, par spectrophotométrie, par le test de capacité absorbante d'oxygène radicalaire (ORAC). Ce test repose sur le fait que la fluorescence de la molécule fluorescéine diminue sous l'action d'un agent oxydant : l'AAPH (« 2,2'-Azobis(2-amidinopropane) dihydrochloride »), qui génère des radicaux peroxyles. La présence d'antioxydants empêche ou ralenti la perte de fluorescence, mesurée, qui est calculée par l'aire sous la courbe en fonction du temps. La capacité antioxydante de l'échantillon testé est ensuite exprimée en équivalent Trolox, c'est-à-dire en fonction de la concentration de Trolox ayant la même activité que l'échantillon testé, à une concentration donnée. La valeur ORAC obtenue est indépendante de la concentration testée pour l'échantillon.

Dans une plaque 96 puits, les quantités suivantes ont été déposées dans chaque puits : 25μl de tampon phosphate (50mM, pH 7.5) pour les blancs ou 25μL de Trolox (25μM, 20μM, 15μM, 10μM, 5μM, 2μM), 25μL d'échantillon dilué, 150μL de Fluorescéine (8nM). La plaque a ensuite été incubée 30 min à 37°C. Juste avant de procéder à la lecture 25μl d'AAPH (153mM) a été ajouté.

Les résultats montrent que les extraits de pépins de raisin ainsi que le mélange d'extraits de *Vitis vinifera* ont des fortes capacité antioxydante avec des valeurs ORAC comprises entre 10524 et 24142. Ainsi un mélange d'extraits de *Vitis vinifera* selon l'invention permet de proposer des formulations avec un fort pourvoir antioxydant. Etant donnée qu'il est recommandé de consommer 3000 à 5000 unités ORAC chaque jour afin de réduire le stress oxydatif, une telle composition selon l'invention permettrait de les atteindre.

## Exemple 11 : Evaluation de l'effet antioxydant d'un mélange d'extraits de *Vitis vinifera* et d'un extrait de reglisse face aux UVB.

L'objectif de cet essai est de comparer la production d'espèces réactives de l'oxygène (ERO) par des kératinocytes suite à une exposition chronique aux UVB en présence d'une composition selon l'invention avec celle d'un contrôle.

Pour ce faire, des fragments de peaux fraîches humaines ont été immédiatement coupés en morceaux de 5 x 5 mm et traités avec de la trypsine-EDTA pendant 3h à 37°C ou pendant une nuit à 4C pour séparer l'épiderme du derme. Les kératinocytes ont été ensemencés à une concentration de $1 \times 10^5$ cellules par $cm^2$ en flasques $75cm^2$ dans du milieu KGM-2, qui comprend de l'hydrocortisone (0,33 μg/ml), le facteur de croissance épidermique EGF (0,125 ng/ml), de l'insuline (5 μg/ml), l'extrait pituitaire bovin (4 μl/ml), de l'épinéphrine (0,39 μg/ml) et de la transferrine (10 μg/ml). Lorsque les cultures ont atteint 70-80 % de confluence, les cellules ont été détachées avec 10% de trypsine-EDTA, puis remises en suspension dans du milieu KGM-2 supplémenté et comptées sur Cellule de Malassez. Les kératinocytes ont été ensuite cultivées à raison de $5 \times 10^4$ cellules par puits en plaque 12 puits pour le test de mesure de production des ERO cytoplasmique via un dérivé de chlorométhyl de H2DCFDA, le CM-H2DCFDA. Trois jours avant irradiation aux UVB, un mélange comprenant une composition selon l'invention a été mis en présence ou non des cellules. Le mélange testé a été le suivant :

- 53.7% d'une composition selon l'invention composée elle-même de 53.4% de E1, et 46.6% de E3 (la composition selon l'invention apportant 19.4% de monomères de flavanols et 28ppm de ε-viniférine) et
- 37.5% d'un extrait de réglisse.

Après changement du milieu de culture, les cellules ont été irradiées ou non une 1ère fois à 60mJ/$cm^2$, puis du

milieu neuf en présence ou non du mélange d'extraits a été remis et les cellules ont été remises à l'étuve à 37°C. Vingt-quatre heures après la 1ère irradiation, le milieu de culture a été changé et les cellules ont été irradiées ou non une 2ème fois à 60mJ/cm$^2$ puis du milieu neuf en présence ou non du mélange d'extraits a été remis et les cellules ont été remises à l'étuve à 37°C. Vingt-quatre heures après la 2ème irradiation, le milieu de culture a été changé et les cellules ont été irradiées ou non une 3ème fois à 60mJ/cm$^2$ puis du milieu neuf en présence ou non du mélange d'extraits a été remis et les cellules ont été remises à l'étuve à 37C pendant 30min.

Trente minutes après la 3ème et dernière irradiation, les cellules ont été détachées des puits de culture par un traitement à la trypsine-EDTA, lavées puis incubées avec 1$\mu$M/tube de CM-H2DCFDA pendant 30 minutes, à 37°C et dans le noir. Après cette incubation, les cellules ont été lavées 2 fois en PBS puis analysées par cytométrie en flux. 1x10$^4$ cellules minimum ont été collectées et analysées par test. Le CM-H2DCFDA permet d'indiquer les dérivés réactifs de l'oxygène (DRO) dans les cellules. Il se diffuse passivement dans les cellules, où ses groupes acétates sont clivés par des estérases intracellulaires et son groupe chlororméthyle réactif aux thiols réagit avec le glutathion intracellulaire et avec d'autres thiols. L'oxydation qui en résulte produit un adduit fluorescent, qui est piégé à l'intérieur de la cellule.

Les valeurs d'intensité de fluorescence moyenne par cellule ont été comparées statistiquement en utilisant une analyse de variance (ANOVA) à deux facteurs ou le facteur 1 était le traitement avec le mélange d'extraits ou non, le facteur 2 était le traitement irradié ou non. Une valeur de $p < 0.05$ est considérée comme significative.

Les résultats montrent qu'une irradiation chronique à 60mJ/cm$^2$ induit un stress oxydant et une production d'ERO significative. En effet l'intensité moyenne de fluorescence par cellule augmente significativement de 1389 à 5346 après expositions aux UVB. En revanche, en présence d'un mélange d'extrait de *Vitis vinifera* et d'un extrait de réglisse, aucune augmentation de l'intensité moyenne de fluorescence n'a été observées, ce qui signifie qu'il n'y a pas eu production cytoplasmique de ERO (Figure 3).

Ces résultats démontrent le pourvoir antioxydant et l'effet protecteur d'un mélange d'extrait de *Vitis vinifera* et d'un extrait de réglisse face à une irradiation aux UVB.

**Exemple 12** : **Evaluation de la viabilité cellulaire suite à une exposition aux UVB et en présence d'un mélange d'extraits de *Vitis vinifera* et d'un extrait de reglisse.**

L'objectif de cet essai est de comparer le taux de prolifération cellulaire des kératinocytes suite à une exposition chronique aux UVB en présence d'une composition selon l'invention avec celle d'un contrôle.

Après isolement des kératinocytes, ceux-ci sont cultivés à raison de 5x10$^3$ cellules par puits en plaque 96 puits. Trois jours avant irradiation aux UVB, un mélange comprenant une composition selon l'invention a été mis en présence ou non des cellules. Le mélange testé a été le suivant :

- 53.7% d'une composition selon l'invention composée elle-même de 53.4% de E1, et 46.6% de E3 (la composition selon l'invention apportant 19.4% de monomères de flavanols et 28ppm de $\epsilon$-viniférine), et
- 37.5% d'un extrait de réglisse.

Après changement du milieu de culture, les cellules ont été irradiées ou non une 1ère fois à 60mJ/cm$^2$, puis du milieu neuf en présence ou non des extraits a été remis et les cellules ont été remises à l'étuve à 37°C. Vingt-quatre heures après la 1ère irradiation, le milieu de culture a été changé et les cellules ont été irradiées ou non une 2ème fois à 60mJ/cm2 puis du milieu neuf en présence ou non des extraits a été remis et les cellules ont été remises à l'étuve à 37°C. Vingt-quatre heures après la 2ème irradiation, le milieu de culture a été changé et les cellules ont été irradiées ou non une 3ème fois à 60mJ/cm$^2$ puis du milieu neuf en présence ou non des extraits a été remis et les cellules ont été remises à l'étuve à 37°C.

Vingt-quatre heures après la 3ème et dernière irradiation, 20$\mu$l de MTS ont été rajoutés dans les puits, puis les cellules ont été remises à l'étuve à 37°C et sous 5% de CO2 pendant 3h. A l'issu des 3h, l'absorbance (DO) a été lue à 490 nm par un spectrophotomètre. Les valeurs de DO sont ensuite exprimées en pourcentage par rapport au contrôle non irradié pour chaque condition.

Les pourcentages de viabilité cellulaire ont été comparées statistiquement en utilisant une analyse de variance (ANOVA) à deux facteurs ou le facteur 1 était le traitement avec le mélange d'extraits ou non, le facteur 2 était le traitement irradié ou non. Une valeur de $p < 0.05$ est considérée comme significative.

Les résultats montrent qu'une irradiation chronique à 60mJ/cm$^2$ induit une diminution significative du nombre de cellules viables. En effet le taux de survie après exposition aux UVB est de 41.5%.

En revanche, en présence d'un mélange d'extrait de *Vitis vinifera* et d'un extrait de réglisse, aucune diminution significative du nombre de cellules vivantes n'a été observée après exposition aux UVB (Figure 4). Ces résultats démontrent l'effet protecteur d'une composition selon l'invention sur la viabilité cellulaire suite à une irradiation aux UVB.

**Exemple 13** : **Exemples de compositions destinées à l'Homme sous différentes formes**

Des exemples de compositions selon l'invention sont présentés dans le tableau 6 ci-dessous.

[Tableau 6]

| Ingrédients | Application Peau | | | Application vision | Application ménopause |
|---|---|---|---|---|---|
| | Exemple 1 | Exemple 2 | Exemple 3 | | |
| Extrait de pépin de raisin | 140 mg | 140 mg | 140 mg | 95 mg | 115 mg |
| Extrait de marc de raisin rouge | 50 mg | 50 mg | 50 mg | 35 mg | 40 mg |
| Extrait de réglisse | 93,5 mg | 93,5 mg | 93,5 mg | | |

| | | | | | |
|---|---|---|---|---|---|
| Vitamine C | 16,5 mg | 16,5 mg | 16,5 mg | | |
| Glutathion | | 50mg | 50mg | | |
| SuperOxyde Dismutase | | | 3,5 mg | | |
| Zinc | | | 16,5 mg | 5 mg | |
| Extrait de safran | | | | 20 mg | 30 mg |
| Vitamine A | | | | 1 mg | |
| Vitamine B6 | | | | | 1 mg |
| **Total** | **300 mg** | **350 mg** | **370 mg** | **156 mg** | **186 mg** |
| Galénique | Gélule | Comprimé | Sachet | Gélule | Gomme à macher |

**Exemple 14** : **Boisson nutritionnelle destinée à l'Homme**

L'exemple 14 est une boisson de 50 ml contenant l'équivalent de 300mg d'un mélange d'extraits de *Vitis vinifera*. Le mélange pouvant être composé de 225mg d'extrait de pépins de raisin et 75mg d'extrait de marc de raisin blanc. Les extraits pourront être microencapsulés. La posologie préconisée est de 1 à 2 shots par jour.

**Exemple 15** : **Huile enrichie en nutriments destinée à l'Homme**

L'exemple 15 est une huile végétale constituée d'huile de pépin de raisin contenant des extraits de *Vitis vinifera* et des caroténoïdes. Une dose quotidienne de 20ml contient 150mg d'un mélange d'extraits de *Vitis vinifera* et 20mg de zéaxanthine.

**Exemple 16** : **Préparation en poudre hyperprotéinée**

L'exemple 16 est une formulation pour denrée alimentaire destinée à des fins médicales spéciales ou une formulation alimentaire enrichie en protéines. La composition est détaillée dans le tableau 7. La posologie peut être par exemple une dose journalière de ladite préparation en poudre. Ladite dose peut-être diluée dans une boisson ou un plat et représente environ 12g.

### Exemple 17 : Yaourt

L'exemple 17 est une formulation sous forme de yaourt, à consommer 2 à 3 fois par jour. La composition est détaillée dans le tableau 7.

[Tableau 7]

| Composition selon l'invention | Exemple 16 | Exemple 17 |
|---|---|---|
| Portion (g) | 100 | 125 |
| | | |
| **Constituants** | | |
| Catéchine+épicatéchine (mg), soit 175mg de B (tableau 3) | 50 | 62.5 |
| ε-viniférine (mg) soit 175mg de B (tableau 3) | 0.0231 | 0.0289 |
| **Autres** | | |
| Protéines (g) | 83 | 10 |
| Vitamine D (μg) | 125 | 15 |
| Vitamine E (mg) | 167 | 20 |
| Vitamine B9 (μg) | 3333 | 400 |
| Vitamine B12 (μg) | 833 | 100 |
| Vitamine C (mg) | 500 | 60 |
| Zinc (mg) | 42 | 5 |

### Exemple 18 : Croquette pour chien

L'exemple 18 est une formulation sous forme de croquettes à destination d'un chien de 10 kg nourri avec 200 g de croquettes/jour apportant :

- 0,450mg de catéchine et/ou épicatéchine par kg de poids corporel, et

- 0,045μg d'ε-viniférine par kg de poids corporel.

**Revendications**

1. Composition comprenant un mélange de molécules comprenant :

- au moins 15% de monomères de flavanols, le pourcentage étant donné en poids sec par rapport au poids sec total de la composition, et
- au moins 15 ppm d'ε-viniférine, en poids sec par rapport au poids sec total de la composition,

ledit mélange de molécules est obtenu à partir d'au moins un extrait ou d'un mélange d'extraits de *Vitis vinifera,* le ratio monomères de flavanols et ε-viniférine est compris entre 20 000 et 4.

2. Composition selon la revendication précédente, ladite composition comprenant au moins 100 ppm de stilbènes comprenant lesdits au moins 15 ppm d'ε-viniferine.

3. Composition selon l'une des précédentes revendications, **caractérisée en ce que** la quantité en poids de monomères de flavanols est supérieure à la quantité en poids d'ε-viniférine.

4. Composition selon l'une des précédentes revendications, **caractérisée en ce qu'**elle comprend des dimères (PAC B1 et B2) de flavanol et **en ce que** la concentration en dimères (PAC B1 et B2) de flavanol est supérieure à 5%, le pourcentage étant donné en poids sec par rapport au poids sec total de la composition.

**5.** Composition selon l'une des revendications précédentes, **caractérisée en ce que** le mélange de molécules est obtenu à partir d'un ou plusieurs produit(s) naturel(s) choisi(s) parmi un extrait de Vitis, un extrait de thé, un extrait de pomme, un extrait de cacao, un extrait d'Iris, de Sophora, de Gnetum, de Carex, de Pivoine ; de Dipterocarpus et un extrait de microalgues.

**6.** Composition selon la revendication précédente, **caractérisée en ce que** l'extrait de *Vitis vinifera* comprend au moins 50% de polyphénols totaux, le pourcentage étant donné en poids sec par rapport au poids total de l'extrait de *Vitis vinifera*.

**7.** Composition selon l'une des précédentes revendications, **caractérisée en ce que** le mélange de molécules est obtenu au moins à partir d'un extrait de marc de raisin et/ou d'un extrait de pépin de raisin.

**8.** Composition selon la revendication précédente, **caractérisée en ce que** le ou les extraits de marc de raisin comprennent des flavonols et **en ce que** la concentration en flavonols dans la composition est supérieure ou égale à 0,05% en poids sec par rapport au poids sec total de la composition.

**9.** Composition selon l'une des revendications précédentes, **caractérisée en ce qu'**elle comprend également un extrait de réglisse.

**10.** Composition selon l'une des revendications précédentes, **caractérisée en ce que** la composition et/ou une ou plusieurs molécules de la composition est (sont) encapsulée(s) ou microencapsulée(s) dans au moins un support alimentaire choisi parmi une maltodextrine, une gomme arabique, une huile hydrogénée, une huile non hydrogénée, une cire, un alginate, l'amidon, une protéine et leurs mélanges.

**11.** Composition selon l'une des revendications précédentes, **caractérisée en ce qu'**elle se présente sous forme de poudre, de gélule, de comprimé, de capsule, d'une solution, d'une suspension, d'une émulsion, d'une gomme à mâcher, d'un produit laitier, de céréales, d'un produit céréalier ou d'une boisson.

**12.** Composition selon l'une des revendications 1 à 11, pour son utilisation comme médicament pour l'Homme ou l'animal.


**Patentansprüche**

**1.** Zusammensetzung, umfassend eine Mischung von Molekülen, umfassend:

- zu mindestens 15 % Flavanolmonomere, wobei der Prozentsatz als Trockengewicht bezogen auf das Gesamttrockengewicht der Zusammensetzung gegeben ist, und
- zu mindestens 15 ppm $\varepsilon$-Viniferin, bezogen auf das Trockengewicht, bezogen auf das Gesamttrockengewicht der Zusammensetzung,
die Molekülmischung aus mindestens einem Extrakt oder einer Mischung von Extrakten von *Vitis vinifera* erhalten wird, wobei das Verhältnis von Flavanolmonomeren und $\varepsilon$-Viniferin zwischen 20.000 und 4 liegt.

**2.** Zusammensetzung nach dem vorstehenden Anspruch, die Zusammensetzung umfassend zu mindestens 100 ppm Stilben, umfassend zu mindestens 15 ppm $\varepsilon$-Viniferin.

**3.** Zusammensetzung nach einem der vorstehenden Ansprüche,
**dadurch gekennzeichnet, dass** die Gewichtsmenge der Flavanolmonomere größer als die Gewichtsmenge von $\varepsilon$-Viniferin ist.

**4.** Zusammensetzung nach einem der vorstehenden Ansprüche,
**dadurch gekennzeichnet, dass** sie Flavanoldimere (PAC B1 und B2) umfasst und dass die Konzentration der Flavanoldimere (PAC B1 und B2) größer als 5 % ist, wobei der Prozentsatz in Trockengewicht bezogen auf das Gesamttrockengewicht der Zusammensetzung gegeben ist.

**5.** Zusammensetzung nach einem der vorstehenden Ansprüche,
**dadurch gekennzeichnet, dass** die Molekülmischung aus einem oder mehreren Naturprodukt(en) gewonnen wird, die aus einem Vitis-Extrakt, einem Tee-Extrakt, einem Apfel-Extrakt, einem Kakao-Extrakt, einem Iris-Extrakt, Sophora, Gnetum, Carex, Pfingstrose; Dipterocarpus und einem Mikroalgen-Extrakt ausgewählt ist.

**6.** Zusammensetzung nach dem vorstehenden Anspruch,
**dadurch gekennzeichnet, dass** der *Vitis-vinifera-Extrakt* zu mindestens 50 % einen Polyphenolgehalt umfasst, wobei der Prozentsatz in Trockengewicht bezogen auf das Gesamtgewicht des *Vitis-vinifera-Extrakts* gegeben ist.

**7.** Zusammensetzung nach einem der vorstehenden Ansprüche,
**dadurch gekennzeichnet, dass** die Molekülmischung mindestens aus einem Traubentresterextrakt und/oder einem Traubenkernextrakt erhalten wird.

**8.** Zusammensetzung nach dem vorstehenden Anspruch,
**dadurch gekennzeichnet, dass** der oder die Traubentresterextrakte Flavonole umfassen und dass die Konzentration der Flavonole in der Zusammensetzung größer als oder gleich 0,05 % Trockengewicht bezogen auf das Gesamttrockengewicht der Zusammensetzung ist.

**9.** Zusammensetzung nach einem der vorstehenden Ansprüche, **dadurch gekennzeichnet, dass** sie auch einen Lakritzextrakt umfasst.

**10.** Zusammensetzung nach einem der vorstehenden Ansprüche, **dadurch gekennzeichnet, dass** die Zusammensetzung und/oder ein oder mehrere Moleküle der Zusammensetzung in mindestens einem Nahrungsmittelträger eingekapselt oder mikroverkapselt ist/sind, der aus einem Maltodextrin, einem Gummiarabikum, einem gehärteten Öl, einem nicht gehärteten Öl, einem Wachs, einem Alginat, einer Stärke, einem Protein und Mischungen davon ausgewählt ist.

**11.** Zusammensetzung nach einem der vorstehenden Ansprüche, **dadurch gekennzeichnet, dass** sie in Form eines Pulvers, einer Hartkapsel, einer Tablette, einer Lösung, einer Suspension, einer Emulsion, eines Kaugummis, eines Milchprodukts, eines Getreides, eines Getreideprodukts oder eines Getränks vorliegt.

**12.** Zusammensetzung nach einem der Ansprüche 1 bis 11, für die Verwendung als Arzneimittel für einen Menschen oder ein Tier.

### Claims

**1.** A composition comprising a mixture of molecules, comprising:

- at least 15% of flavanol monomers, the percentage being given by dry weight relative to the total dry weight of the composition, and
- at least 15 ppm of $\varepsilon$-viniferin, by dry weight relative to the total dry weight of the composition,
said mixture of molecules is obtained from at least one extract or a mixture of extracts of *Vitis vinifera,* the ratio of flavanol monomers to $\varepsilon$-viniferin is between 20,000 and 4.

**2.** The composition according to the preceding claim, said composition comprising at least 100 ppm of stilbenes comprising said at least 15 ppm of $\varepsilon$-viniferin.

**3.** The composition according to either of the preceding claims, **characterized in that** the amount by weight of flavanol monomers is greater than the amount by weight of $\varepsilon$-viniferin.

**4.** The composition according to any of the preceding claims,
**characterized in that** it comprises flavanol dimers (PAC B1 and B2) **and in that** the concentration of flavanol dimers (PAC B1 and B2) is greater than 5%, the percentage being given by dry weight relative to the total dry weight of the composition.

**5.** The composition according to any of the preceding claims,
**characterized in that** the mixture of molecules is obtained from one or more natural product(s) selected from a Vitis extract, a tea extract, an apple extract, a cocoa extract, an iris, Sophora, Gnetum, Carex, peony, Dipterocarpus extract and a microalgae extract.

**6.** The composition according to the preceding claim, **characterized in that** the *Vitis vinifera* extract comprises at least 50% total polyphenols, the percentage being given by dry weight relative to the total weight of the *Vitis vinifera* extract.

7. The composition according to any of the preceding claims, **characterized in that** the mixture of molecules is obtained at least from one grape pomace extract and/or from a grape seed extract.

8. The composition according to the preceding claim, **characterized in that** the grape pomace extract(s) comprise flavonols **and in that** the concentration of flavonols in the composition is greater than or equal to 0.05% by dry weight relative to the total dry weight of the composition.

9. The composition according to any of the preceding claims, **characterized in that** it also comprises a licorice extract.

10. The composition according to any of the preceding claims, **characterized in that** the composition and/or one or more molecules of the composition is (are) encapsulated or microencapsulated in at least one food support selected from a maltodextrin, a gum arabic, a hydrogenated oil, a non-hydrogenated oil, a wax, an alginate, starch, a protein and mixtures thereof.

11. The composition according to any of the preceding claims, **characterized in that** it is in the form of a powder, gelcap, tablet, capsule, solution, suspension, emulsion, chewing gum, dairy product, cereals, cereal product or beverage.

12. The composition according to any of claims 1 to 11, for use thereof as a drug for humans or animals.

**Figure 1**

**Figure 2**

**Figure 3**

**Figure 4**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2018271928 A, Gyongyi Nemeth
- FR 3042712, Al-Awwadi Najim a

**Littérature non-brevet citée dans la description**

- **NAIBERG**. *Psychosom Med*, 2016, vol. 78 (2), 192-207
- **FOUDA et al.** *Arterioscler Thromb Vasc Biol*, April 2019, vol. 39 (4), 593-602
- **SINGH et al.** *Eur J Ophthalmol*, 27 April 2020, 1120672120914232
- **CIBOR et al.** *World J Gastroenterol*, 21 January 2016, vol. 22 (3), 1067-1077
- **KOVÂCS I et al.** *J.Cardiovasc Pharmacol*, February 2008, vol. 51 (2), 148-53
- **SOMANI YB et al.** *Am J Physiol Heart Circ Physiol*, 01 August 2019, vol. 317 (2), H395-H404
- **TAKASE B et al.** *Clin Cardiol*, April 2004, vol. 27 (4), 223-7
- **BUDHIRAJA et al.** *J Clin Sleep Med*, 15 June 2007, vol. 3 (4), 409-15
- **WOLF et al.** *Exp Physiol*, 2019, vol. 104 (7), 1136-1146
- **SHAFABAKHSH et al.** *Crit Rev Food Sci Nutr*, 2020, vol. 60 (14), 2369-2378
- **HASKELL-RAMSAY et al.** *Nutrients*, 27 July 2018, vol. 10 (8), 986
- **CICERO et al.** *Arch Med Sci*, 2019, vol. 15 (4), 936-943
- **WU et al.** *The Kaohsiung Journal of Medical Sciences*, 2020, vol. 36, 535-542
- **JAYAPRAKASHA et al.** *Antioxidant activity of grape seed (Vitis vinifera) extracts on peroxidation models in vitro*, 01 May 2001
- **BENSALEM JULIEN et al.** *Polyphenols From Grape and Blueberry Improve Episodic Memory in Healthy Elderly with Lower Level of Memory Performance: A Bicentric Double-Blind, Randomized, Placebo-Controlled Clinical Study*, 18 June 2019
- **PIERRE PHILIP et al.** *Acute Intake of a Grape and Blueberry Polyphenol-Rich Extract Ameliorates Cognitive Performance in Healthy Young Adults During a Sustained Cognitive Effort*, 17 December 2019
- **YUN CHEONG-YONG et al.** *alpha]-Viniferin Improves Facial Hyperpigmentation via Accelerating Feedback Termination of cAMP/PKA-Signaled Phosphorylation Circuit in Facultative Melanogenesis*, 01 January 2018
- **KAZUOMI SATO et al.** *of Catechins*, 04 November 2009
- **ZOLGHADRI SAMANEH et al.** *A comprehensive review on tyrosinase inhibitors*, 01 January 2019
- **LIKHITWITAYAWUID et al.** *Stilbenes with tyrosinase inhibitory activity*, 10 January 2008
- **FIORE et al.** *J Ethnopharmacol*, 14 July 2005, vol. 99 (3), 317-24